# FASCICULE DE BREVET EUROPEEN

(11) **EP 2 999 962 B1**
(45) Date de publication et mention de la délivrance du brevet: **01.02.2017**
(21) Numéro de dépôt: 14731694.7
(22) Date de dépôt: 20.05.2014
(51) Int. Cl.: G01N 31/02

(54) **PROCÉDÉ POUR LA DÉTECTION, LA CAPTURE ET/OU LE RELARGAGE D'ÉLÉMENTS CHIMIQUES**
VERFAHREN ZUR ERKENNUNG, AUFNAHME UND/ODER FREISETZUNG CHEMISCHER ELEMENTE
METHOD FOR DETECTING, CAPTURING AND/OR RELEASING CHEMICAL ELEMENTS

(30) Priorité: 20.05.2013 FR 1354510
(43) Date de publication de la demande: 30.03.2016
(73) Titulaire: Université d'Aix-Marseille, 13007 Marseille (FR); Centre National de la Recherche Scientifique (C.N.R.S.), 75016 Paris (FR); Ecole Centrale de Marseille, 13013 Marseille (FR); Université Claude Bernard Lyon 1 (UCBL), 69100 Villeurbanne (FR)
(72) Inventeur: LECLAIRE, Julien, 13013 Marseille (FR); CANARD, Gabriel, 13004 Marseille (FR); FOTIADU, Frédéric, 13008 Marseille (FR); POISSON, Guillaume, 13013 Marseille (FR)
(74) Mandataire: Cabinet Becker et Associés
(86) Numéro de dépôt international: PCT/FR2014/051172
(87) Numéro de publication internationale: WO 2014/188115

(56) Documents cités:
- FR-A1- 2 969 504
- JULIEN LECLAIRE ET AL: "CO 2 Binding by Dynamic Combinatorial Chemistry: An Environmental Selection", JOURNAL OF THE AMERICAN CHEMICAL SOCIETY, vol. 132, no. 10, 17 mars 2010 (2010-03-17), pages 3582-3593, XP055109495, ISSN: 0002-7863, DOI: 10.1021/ja909975q cité dans la demande
- GABRIEL CANARD ET AL: "Effective Concentration as a Tool for Quantitatively Addressing Preorganization in Multicomponent Assemblies: Application to the Selective Complexation of Lanthanide Cations", JOURNAL OF THE AMERICAN CHEMICAL SOCIETY, vol. 130, no. 3, 1 janvier 2008 (2008-01-01), pages 1025-1040, XP055109559, ISSN: 0002-7863, DOI: 10.1021/ja0772290
- KOEN BINNEMANS ET AL: "Recycling of rare earths: a critical review", JOURNAL OF CLEANER PRODUCTION, vol. 51, 5 janvier 2013 (2013-01-05), pages 1-22, XP055109592, ISSN: 0959-6526, DOI: 10.1016/j.jclepro.2012.12.037

## Description

La présente invention concerne un procédé adapté pour la détection, la capture et/ou le relargage sélectif d'éléments chimiques choisis parmi les métaux pauvres, les alcalins, les alcalino-terreux, les actinides et les terres rares. Ces procédés mettent en oeuvre un assemblage moléculaire formé d'au moins une amine, au moins un aldéhyde et/ou une imine et/ou du CO₂, ou un adduit formé par la mise en contact d'une amine et de CO₂, et au moins un de ces éléments chimiques. L'invention concerne également un kit pour la mise en oeuvre du procédé de détection, capture et/ou relargage.

### Introduction

Certains éléments chimiques, comme les actinides et les terres rares, sont largement utilisés de nos jours pour de nombreuses applications, par exemple dans les batteries, les aimants, ou les luminaires à basse consommation. Même si les terres rares sont des éléments présents en quantité abondante dans l'écorce terrestre, la demande mondiale de ces éléments pour les nouvelles technologies, en augmentation croissante, et le monopole de la Chine sur la production en font des éléments stratégiques.

Les concentrés de terres rares en mélange sont obtenus après plusieurs étapes de séparation des minerais extraits pour obtenir des niveaux de pureté très élevés. Des concentrés de terres rares peuvent aussi être obtenus à partir de déchets post-consommation par des recycleurs spécialisés. Dans ce cadre, il existe une préoccupation croissante concernant les possibilités de séparer ces éléments entre eux ou d'autres éléments. A ce jour, les procédés de tri sélectif de ce type d'éléments reposent principalement sur des procédés d'extraction liquide/liquide à contre-courant, après que la chromatographie ionique et la précipitation sélective ont été abandonnées. Ces méthodes sont coûteuses en termes d'infrastructures, de temps et d'énergie, avec des capacités et des performances faibles.

Une nouvelle approche de séparation susceptible de réduire considérablement les coûts d'opération et d'infrastructures par rapport à la technologie de séparation liquide-liquide utilisée dans l'industrie des terres rares a été mise au point. Son principe se base sur un article récemment publié qui décrit l'auto-assemblage de briques moléculaires spécifiques en présence de CO₂ (Leclaire et al. J. Am. Chem. Soc. 2010, 132, p.3582-3593), et envisage l'utilisation de ces assemblages moléculaires pour séparer différents composants d'un mélange.

De façon inattendue, il a été mis en évidence que des systèmes analogues à ceux décrits dans l'article ci-dessus, mettant en jeu des briques organiques simples, permettent également la formation d'une architecture sophistiquée impliquant des éléments chimiques choisis parmi les métaux pauvres, les alcalins, les alcalino-terreux, les actinides et les terres rares. La formation de ces assemblages conduit à leur précipitation, et permet ainsi d'envisager leur utilisation dans des procédés de détection, capture et/ou relargage des éléments chimiques précités peu coûteux, simples à mettre en oeuvre et pouvant utiliser un déchet néfaste pour l'environnement (le CO₂).

### Résumé de l'invention

Un premier objet de l'invention est un procédé de détection et/ou capture d'au moins un élément chimique choisi parmi les métaux pauvres, les alcalins, les alcalino-terreux, les actinides et les terres rares dans un échantillon, comprenant la mise en contact de :
- au moins une amine et au moins un aldéhyde et/ou une imine (formée par la mise en contact d'un aldéhyde et une amine) et/ou du CO₂, ou
- un adduit formé par la mise en contact d'une amine et de CO₂,
avec au moins un échantillon susceptible de comprendre au moins un desdits éléments chimiques. La mise en contact de ces composants conduit à la formation d'un assemblage moléculaire, permettant la détection et/ou la capture du ou des éléments chimiques désirés lorsqu'ils sont présents.

Un second objet de l'invention est un procédé de relargage d'au moins un élément chimique choisi parmi les métaux pauvres, les alcalins, les alcalino-terreux, les actinides et les terres rares comprenant la séparation, au moins partielle, de préférence totale, des composants d'un assemblage formé par la mise en contact de :
- au moins une amine et au moins un aldéhyde et/ou une imine et/ou du CO₂, ou
- un adduit formé par la mise en contact d'une amine et de CO₂,
avec un échantillon comprenant ledit ou lesdits éléments chimiques.

Un troisième objet de l'invention est un procédé de formation d'un assemblage moléculaire comprenant la mise en contact de :
- au moins une amine et au moins un aldéhyde et/ou une imine (formée par la mise en contact d'un aldéhyde et une amine) et/ou du CO₂, ou
- un adduit formé par la mise en contact d'une amine et de CO₂,
avec au moins un échantillon comprenant au moins un élément chimique choisi parmi les métaux pauvres, les alcalins, les alcalino-terreux, les actinides et les terres rares.

Un dernier objet de l'invention est un kit pour la détection, la capture et/ou le relargage d'au moins un élément chimique choisi parmi les métaux pauvres, les alcalins, les alcalino-terreux, les actinides et les terres rares, comprenant au moins une amine, et au moins un aldéhyde et/ou une imine et/ou du CO₂, ou un adduit formé par la mise en contact d'une amine et de CO₂.

### Description des figures

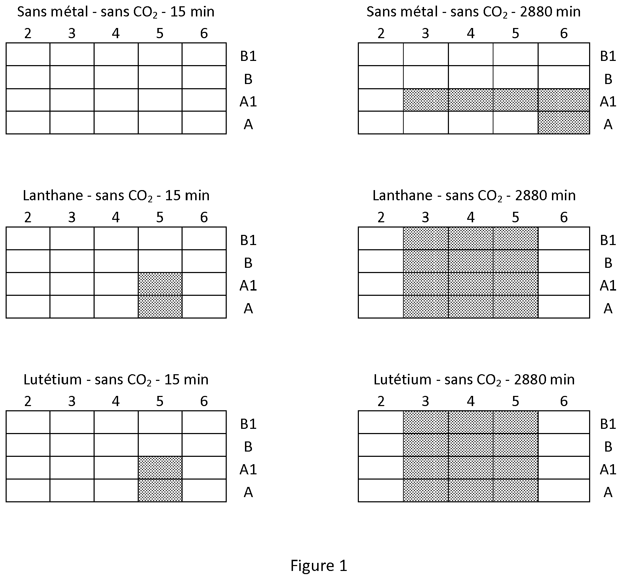

Figure 1 : Analyse des bibliothèques réalisées sans ajout de CO₂, après 15 minutes et 2880 minutes. Chaque case correspond à la combinaison d'une amine en abscisse et d'un aldéhyde ou une imine en ordonnée. Une case grisée correspond à l'observation d'un précipité prouvant la formation d'un assemblage.

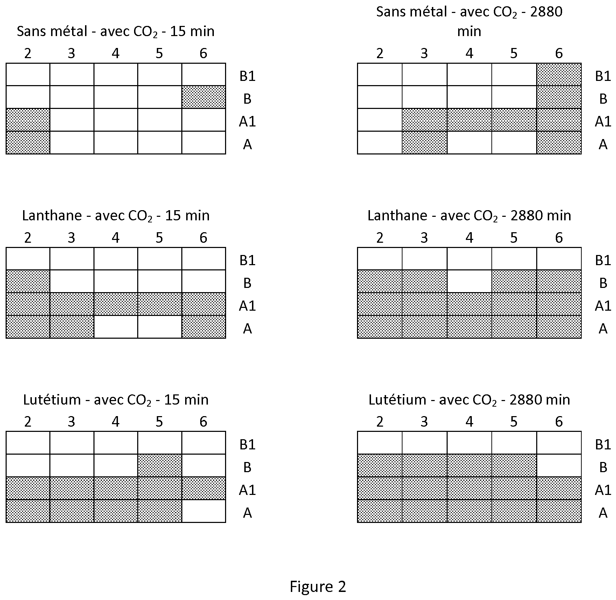

Figure 2 : Analyse des bibliothèques réalisées en présence de CO₂, après 15 minutes et 2880 minutes. Chaque case correspond à la combinaison d'une amine en abscisse et d'un aldéhyde ou une imine en ordonnée. Une case grisée correspond à l'observation d'un précipité prouvant la formation d'un assemblage.

### Description détaillée de l'invention

Les procédés de détection, de capture et/ou de relargage des éléments chimiques selon l'invention sont basés sur la formation d'assemblages moléculaires lors de la mise en contact des éléments chimiques avec au moins une amine, et au moins un aldéhyde et/ou une imine et/ou du CO₂, ou avec un adduit formé par la mise en contact d'une amine et de CO₂. La formation d'un tel assemblage est détectée par la formation d'un précipité au sein du milieu comprenant les différents composants.

Dans un mode de réalisation, l'invention concerne un_procédé de détection et/ou capture d'au moins un élément chimique choisi parmi les alcalins, les alcalino-terreux, les actinides et les terres rares dans un échantillon, comprenant la mise en contact d'au moins une amine, au moins un aldéhyde et/ou une imine, éventuellement du CO₂, et éventuellement un adduit formé par la mise en contact d'une amine et de CO₂, et au moins un desdits éléments chimiques.

### Description des différents composants des assemblages selon l'invention

### - Aldéhyde

Un aldéhyde selon l'invention est un composé comportant au moins une fonction aldéhyde CHO. Ainsi, l'aldéhyde peut être de formule générale

(I) R1-CHO,

dans laquelle R1 est choisi parmi les groupes alkyles, alcényles, acynyles, cycloalkyles, cycloalcényles, cycloalcynyles et les groupes aromatiques, dont la chaîne hydrocarbonée est éventuellement interrompue par au moins un hétéroatome choisi parmi N, O et S, et éventuellement substitués par au moins un substituant. De préférence, les substituants ne comprennent indépendamment pas d'amine (-NHR ou - NH₂), d'hydrazine (-NH-NH₂), d'hydrazide (C(O)-NH-NH₂), ni d'hydroxylamine (-O-NH₂). De préférence, les substituants sont tels que définis ci-dessous.

De préférence, l'aldéhyde est de formule générale 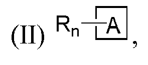 dans laquelle 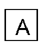 représente un cycle hydrocarboné choisi parmi les cycloalkyles, les cycloalcènes, les cycloalcynes et les aromatiques, comportant n chaînons, n étant compris entre 5 et 7, de préférence n vaut 5 ou 6. En particulier, le cycle hydrocarboné est un phényle. Le cycle hydrocarboné peut éventuellement être interrompu par au moins un hétéroatome choisi parmi N, O et S. Chaque substituant Rₙ est choisi parmi un atome d'hydrogène, un aldéhyde CHO, un groupe hydroxyle -OH, un thiol -SH, un atome d'halogène, un groupe alkoxyle, et un alkyle en C₁-C₂₀, de préférence en C₁-C₃. Dans un mode de réalisation préférentiel, chaque groupe alkyle ne comprend indépendamment aucun substituant amine (-NHR ou -NH₂), hydrazine (-NH-NH₂), hydrazide (C(O)-NH-NH₂), ni hydroxylamine (-O-NH₂). Au moins un Rₙ comprend, de préférence est, un aldéhyde CHO.

Dans la présente invention, un groupe alkyle désigne un groupe hydrocarboné, linéaire ou ramifié en C₁-C₂₀, de préférence en C₁-C₆, en particulier en C₁-C₃. De préférence, le groupe alkyle est choisi parmi les groupes méthyle, éthyle, n-propyle, isopropyle, n-butyle, sec-butyle, tert-butyle, n-pentyle et n-hexyle. Le groupe alkyle peut éventuellement être interrompu par au moins un hétéroatome choisi parmi N, O et S. Le groupe alkyle peut éventuellement être substitué, notamment par au moins un groupe choisi parmi les hydroxyles (-OH), les alkoxyles (-OR), les thiols (-SH), les thioéthers (-SR), les carbonyles (-CHO ou -C(O)R), les carboxyles (-COOH ou -COOR), les amines -NH₂, les hydrazines (-NH-NH₂), les hydrazides (C(O)-NH-NH₂), les hydroxylamines (-O-NH₂), les halogènes, les imines (-CH=NR), les oximes (-CH=NOR ou -CH=NOH), les hydrazones (-CH=N-NHR), les acylhydrazones (-CH=N-NH-C(O)-R), les aminals (-CH(NHR)-NHR'), les oxazolidines (-CH(OR)-NHR') et les thiazolidines (-CH(SR)-NHR'), où R et R' sont de préférence indépendamment des groupes alkyles hydrocarbonés non substitués et comprenant uniquement des liaisons simples.

Un groupe alcényle désigne un groupe alkyle, tel que défini ci-dessus, comprenant en outre au moins une double liaison C=C.

Un groupe alcynyle désigne un groupe alkyle, tel que défini ci-dessus, comprenant en outre au moins une triple liaison C≡C.

Les groupes cycloalkyles, cycloalcényles et cycloalcynyles représentent respectivement des groupes alkyles, alcényles et alcynyles cycliques.

Un aromatique est un groupe comprenant au moins un cycle plan comportant un système π conjugué, formé de liaisons doubles et/ou de doublets non-liants, dans lequel chaque atome du cycle comporte une orbitale p, les orbitales p se recouvrent, et la délocalisation des électrons π entraîne une diminution de l'énergie de la molécule. De préférence, un aromatique est choisi parmi les groupes phényle, pyridinyle, pyrimidinyle, pyrazinyle, triazinyle, furanyle, thiophényle, pyrrolyle, imidazolyle, thiazolyle, oxazolyle et naphtyle. De façon hautement préférée, le groupe aromatique est un phényle.

Un groupe alkoxyle (-O-alkyle) désigne un groupe alkyle tel que défini précédemment, ledit groupe alkyle étant relié au reste de la molécule par l'intermédiaire d'un atome d'oxygène.

Un halogène désigne un atome choisi parmi le fluor, le brome, le chlore et l'iode, de préférence le chlore ou le brome.

De préférence, l'aldéhyde est de formule (III) 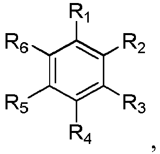 dans laquelle au moins un parmi R₁ à R₆ est un groupe aldéhyde, et les autres sont choisis parmi des atomes d'hydrogène, des groupes alkyles en C₁-C₂₀, des groupes hydroxyles, des groups thiols, des atomes d'halogène, et des groupes alkoxyles en C₁-C₂₀.

Dans un mode de réalisation préféré, l'aldéhyde est choisi parmi les composés suivants :

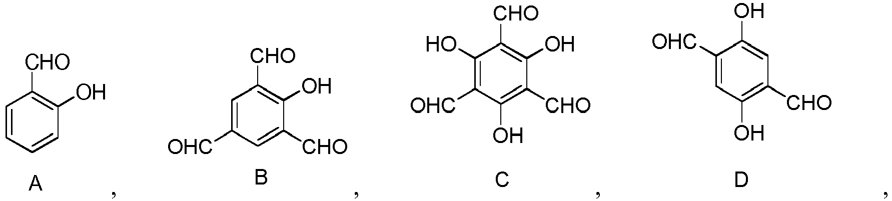

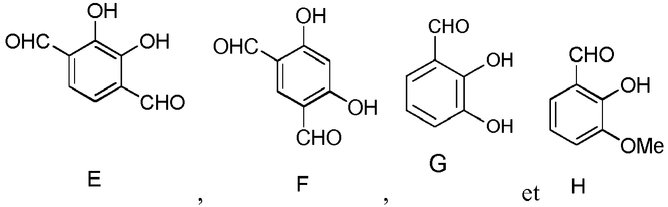

De façon hautement préféré, l'aldéhyde est choisi parmi les composés B et C, en particulier l'aldéhyde est le composé C.

Les aldéhydes selon l'invention peuvent être obtenus par toute voie de synthèse adaptée connue dans l'art. Ainsi, par exemple, certains aldéhydes de l'invention peuvent être obtenus par réaction de Duff avec l'hexaméthylènetétramine.

### - Amine

Une amine selon l'invention est un composé comprenant au moins une, de préférence une ou deux, fonction amine primaire -NH₂ ou secondaire -NHR, où R est tel que précédemment décrit, et éventuellement au moins une fonction amine tertiaire. L'amine selon l'invention peut être de formule générale (IV) R2-NH-R3, dans laquelle R2 est choisi parmi les groupes alkyles, alcényles, acynyles, cycloalkyles, cycloalcényles, cycloalcynyles et les groupes aromatiques, dont la chaîne hydrocarbonée est éventuellement interrompue par au moins un hétéroatome choisi parmi N, O et S, et éventuellement substitués par au moins un substituant tel que défini ci-dessus, lequel substituant ne comprend de préférence pas d'aldéhyde CHO ; R3 est choisi parmi un atome d'hydrogène, les groupes alkyles, alcényles, alcynyles, cycloalkyles, cycloalcényles, cycloalcynyles et les groupes aromatiques, dont la chaîne hydrocarbonée est éventuellement interrompue par au moins un hétéroatome choisi parmi N, O et S, et éventuellement substitués par au moins un substituant tel que défini ci-dessus. De préférence, les substituants ne comprennent indépendamment pas d'aldéhyde CHO.

Dans un mode de réalisation, l'amine comprend une seule fonction amine.

Dans un second mode de réalisation, l'amine comprend au moins 2 fonctions amines, de préférence au moins 3, au moins 4, ou au moins 5 fonctions amines. En particulier, l'amine est telle que R3 est un atome d'hydrogène et R2 est un groupe alkyle dont la chaîne hydrocarbonée est interrompue par au moins un atome d'azote.

Dans un mode de réalisation particulier, le procédé selon l'invention comprend la mise en contact notamment d'une amine comprenant au moins 2 fonctions amines, et d'une imine formée par réaction d'un aldéhyde avec une amine comprenant une seule fonction amine.

Dans un autre mode de réalisation particulier, le procédé selon l'invention comprend la mise en contact notamment d'une amine comprenant au moins 2 fonctions amines, et d'un adduit formé par réaction d'une amine comprenant une seule fonction amine avec le CO₂.

De préférence, une amine selon l'invention est choisie parmi les composés suivants : 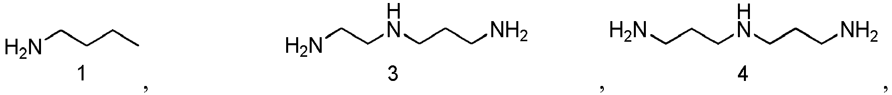 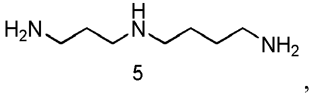 l' éthylènediamine 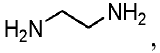 la tris-(2-aminoéthyl)amine 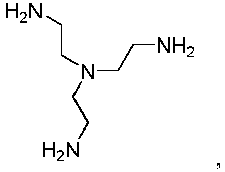 une polyéthylèneimine, en particulier une polyéthylèneimine linéaire ou ramifiée, notamment la diéthylènetétramine 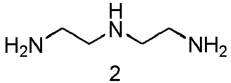 ou la triéthylènetétramine 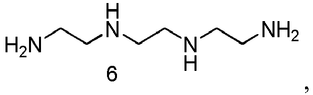 la pipérazine 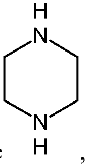 la cis-1,4-cyclohexanediamine 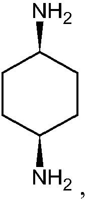 la trans-1,4-cyclohexanediamine 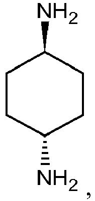 et la phénylènediamine

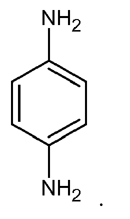

Dans un mode de réalisation préféré, l'amine est choisie parmi l'éthylènediamine, la diéthylènetriamine 2, la triéthylènetétramine 6 et la tris-(2-aminoéthyl)amine.

De façon hautement préférée, l'amine est le composé 2.

Les aldéhydes et amines de l'invention sont choisis par l'homme du métier en fonction de leurs caractéristiques structurales, par exemple du nombre de fonctions réactives (aldéhydes et/ou amines) et/ou de la masse molaire. La formation des assemblages implique la formation de liaisons imines, par réaction des groupes amines des amines avec les groupes aldéhydes des aldéhydes. Des liaisons de type hydrazone, oxime, aminal, oxazolidine, thiazolidine et/ou carbamate peuvent être également mises enjeu.

### - Imine

Une imine selon l'invention est obtenue par mise en contact d'un aldéhyde selon l'invention avec une amine de formule (IV) telle que définie ci-avant. De préférence, l'amine comprend une seule fonction amine. De préférence, R2 est un groupe alkyle hydrocarboné non substitué et comprenant uniquement des liaisons simples et R3 est un atome d'hydrogène, en particulier l'amine est la n-butylamine. Selon un mode de réalisation de l'invention, tous les groupes CHO de l'aldéhyde selon l'invention réagissent avec l'amine. Selon un autre mode de réalisation de l'invention, seulement certains groupes CHO de l'aldéhyde réagissent avec l'amine. Ainsi, une imine selon l'invention peut comprendre à la fois des groupes CHO et des groupes imines.

De préférence, une imine selon l'invention est choisie parmi les composés suivants : 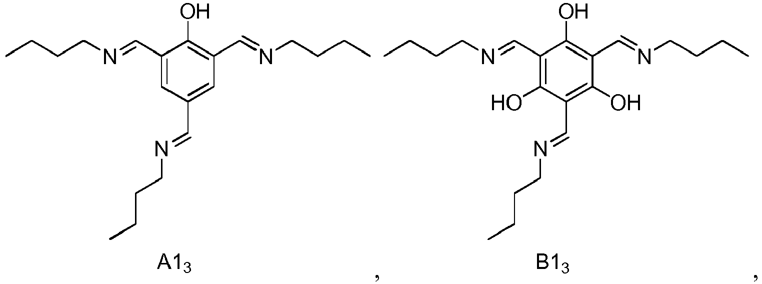 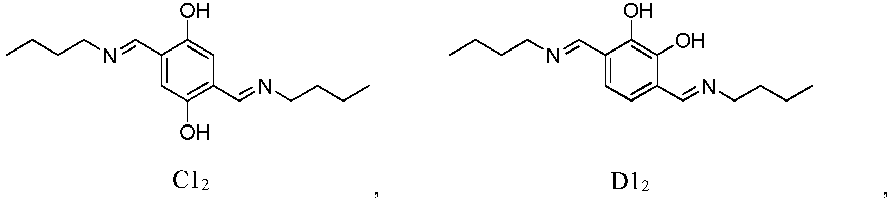 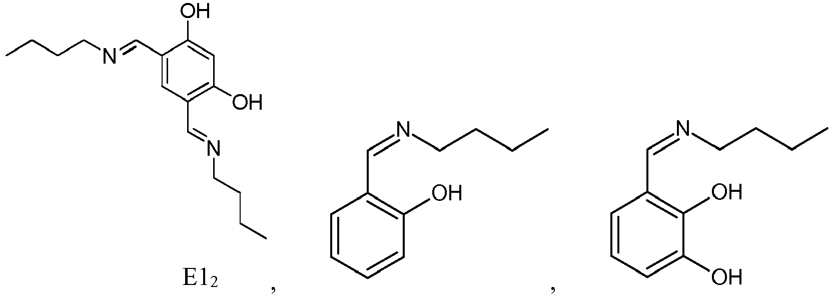 et

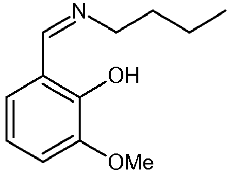

De façon hautement préféré, l'imine est choisie parmi les composés A1₃ et B1₃, en particulier la polyimine est le composé B1₃.

### - Adduit formé par la mise en contact d'une amine et de CO₂

Un adduit selon l'invention est susceptible d'être obtenu, de préférence il est obtenu, par mise en contact d'une amine selon l'invention avec du dioxyde de carbone. L'adduit peut être un carbamate. Selon l'amine utilisée, l'adduit peut également être un aminal (si l'amine est une éthanediamine ou une propanediamine), une oxazolidine (si l'amine est un aminoéthanol ou un aminopropanol), ou une thiazolidine (si l'amine est un aminoéthanethiol ou un aminopropanethiol).

### - Dioxyde de carbone

Un assemblage formé selon le procédé de l'invention peut éventuellement comprendre du dioxyde de carbone. Le dioxyde de carbone utilisé peut être issu d'une activité humaine dont il représente un déchet, par exemple il peut provenir de fumées de combustion, de gaz de raffinerie, de gaz de cémenterie ou de gaz de hauts-fourneaux.

### - Elément chimique

L'assemblage formé selon le procédé de l'invention comprend au moins un élément chimique choisi parmi les métaux pauvres, les alcalins, les alcalino-terreux, les actinides et les terres rares.

Dans le cadre de la présente invention, métaux pauvres désignent les éléments métalliques du groupe p du tableau périodique. Le groupe des métaux pauvres comprend l'Aluminium ₁₃Al, le Gallium ₃₁Ga, l'Indium ₄₉In, l'Étain ₅₀Sn, le Thallium ₈₁Tl, le Plomb ₈₂Pb, le Bismuth ₈₃Bi, le Polonium ₈₄Po et le Flérovium ₁₁₄Fl. De préférence, le métal pauvre est l'indium.

Dans le cadre de la présente invention, les alcalins, ou métaux alcalins, désignent les éléments de la première colonne du tableau périodique, à l'exception de l'hydrogène. Le groupe des alcalins comprend le lithium ₃Li, le sodium ₁₁Na, le potassium ₁₉K, le rubidium ₃₇Rb, le césium ₅₅Cs et le francium ₈₇Fr. De préférence, le métal alcalin est le césium.

Dans le cadre de la présente invention, les alcalino-terreux désignent les éléments de la deuxième colonne du tableau périodique. Le groupe des alcalino-terreux comprend le béryllium ₄Be, le magnésium ₁₂Mg, le calcium ₂₀Ca, le strontium ₃₈Sr, le baryum ₅₆Ba et le radium ₈₈Ra.

Dans le cadre de la présente invention, les actinides désignent l'actinium ₈₉Ac, le thorium ₉₀Th, le protactinium ₉₀Pa, l'uranium ₉₂U, le neptunium ₉₃Np, le plutonium ₉₄Pu, l'américium ₉₅Am, le curium ₉₆Cm, le berkélium ₉₇Bk, le californium ₉₈Cf, l'einsteinium ₉₉Es, le fermium ₁₀₀Fm, le mendélévium ₁₀₁Md, le nobelium ₁₀₂No, et le lawrencium ₁₀₃Lr. De préférence, les actinides sont choisis parmi le thorium et l'uranium.

Dans le cadre de la présente invention, les terres rares désignent le scandium ₂₁ Sc, l'yttrium ₃₉Y et les quinze lanthanides. Les lanthanides désignent le lanthane ₅₇La, le cérium ₅₈Ce, le praséodyme ₅₉Pr, le néodyme ₆₀Nd, le prométhium ₆₀Pm, le samarium ₆₂Sm, l'europium ₆₃Eu, le gadolinium ₆₄Gd, le terbium ₆₅Tb, le dysprosium ₆₆Dy, l'holmium ₆₇Ho, l'erbium ₆₈Er, le thulium ₆₉Tm, l'ytterbium ₇₀Yb et le lutétium ₇₁Lu. De préférence, les terres rares comprennent le lanthane et/ou le lutétium.

Selon un mode de réalisation particulier, au moins un des éléments chimiques est une (ou des) terre(s) rare(s), en particulier La et/ou Lu.

L'élément chimique peut être mis en contact avec les autres composants sous la forme d'élément sans liaison à un autre atome, ou sous la forme d'un dérivé, tel qu'un sel ou un complexe. Par exemple, l'élément peut être mis en contact avec les autres composants sous la forme d'un nitrate, d'un trifluoroacétate ou d'un acétate.

L'échantillon sur lequel le procédé de détection et/ou capture selon l'invention est mis en oeuvre peut être n'importe quel type d'échantillon susceptible de comprendre un ou plusieurs éléments chimiques tels que définis ci-dessus. Il peut s'agir d'un échantillon de toute origine.

Les quantités (absolues ou relatives) des différents composants de l'assemblage formé selon le procédé de l'invention peuvent être adaptées par l'homme du métier de sorte à optimiser la formation de l'assemblage. En particulier, l'homme du métier est à même d'ajuster les proportions relatives d'aldéhyde et d'amine, dans le cas où ces deux types de composants font partie de l'assemblage désiré, de sorte que le rapport entre le nombre de fonctions aldéhydes et le nombre de fonctions amines mises en présence soit compris entre environ 0,1 et environ 10, de préférence compris entre environ 0,8 et environ 1,2, en particulier environ égal à 1.

Dans un mode de réalisation particulier, le rapport entre le nombre de moles d'amine et le nombre de moles de l'élément que l'on cherche à détecter et/ou à capturer est supérieur ou égal à 6, de préférence supérieur ou égal à 12. Sans vouloir être lié par une quelconque théorie, il semble qu'un tel rapport permette de conférer des propriétés de réversibilité thermique au précipité qui se forme en présence de l'élément chimique. Ainsi, il est possible dans ce cas de relarguer l'élément chimique selon le procédé de relargage décrit plus loin par simple chauffage, de préférence à une température inférieure ou égale à 100°C.

Les valeurs de rapport entre le nombre de moles d'amine et le nombre de moles de l'élément que l'on cherche à détecter et/ou à capturer permettant d'obtenir une telle réversibilité thermique peuvent éventuellement varier en fonction du contre-ion de l'élément chimique que l'on cherche à détecter et/ou à capturer, tel que le nitrate, l'acétate, ou le trifluoroacétate.

Dans la présente invention, le terme « environ » relatif à une valeur numérique désigne un intervalle compris entre moins et plus 10% de ladite valeur numérique.

### Procédé de détection et/ou capture des éléments chimiques

Le procédé de détection et/ou capture des éléments chimiques selon l'invention comprend la mise en contact de :
- au moins une amine et au moins un aldéhyde et/ou une imine (formée par la mise en contact d'un aldéhyde et une amine) et/ou du CO₂, ou
- un adduit formé par la mise en contact d'une amine et de CO₂,
avec au moins un échantillon susceptible de comprendre au moins un élément chimique choisi parmi les métaux pauvres, les alcalins, les alcalino-terreux, les actinides et les terres rares.

De préférence, le procédé comprend la mise en contact d'au moins une amine et de CO₂, ou d'un adduit formé par la mise en contact d'une amine et de CO₂, avec ledit échantillon.

La mise en contact peut être effectuée par toute technique adaptée et connue dans l'art. De préférence, la mise en contact est réalisée à température ambiante, c'est-à-dire comprise entre environ 15 et environ 25°C.

Le procédé peut éventuellement comprendre en outre une étape de chauffage du mélange obtenu à une température plus élevée que celle de la mise en contact, de préférence à une température comprise entre 26 et 100°C, en particulier à une température voisine de 90°C. Dans ce cas, le procédé comprend également ensuite une étape de refroidissement contrôlé du mélange jusqu'à température ambiante telle que définie ci-dessus.

Dans ce cas, un précipité peut être obtenu lors de la mise en contact à température ambiante et/ou lors du refroidissement en fin de procédé.

L'homme du métier est à même de choisir la température et la vitesse de refroidissement adaptées pour mettre en oeuvre le procédé en fonction notamment de la nature chimique des composants de l'assemblage et des critères de stabilité thermodynamique et/ou cinétique de celui-ci. Ainsi, pour obtenir une précipitation dans des conditions thermodynamiques et pas cinétiques, il peut être intéressant de chauffer le milieu, puis de le refroidir lentement. Sans vouloir être liés par une quelconque théorie, il semble qu'une meilleure sélectivité et une meilleure réversibilité puissent être généralement obtenues avec les précipités obtenus dans des conditions thermodynamiques. A haute température, les échanges sont favorisés et l'assemblage peut s'auto-organiser selon l'agencement le plus stable si la descente en température est lente, ce qui devrait permettre également de capturer de plus grandes quantités d'éléments chimiques.

Une mise en contact peut être réalisée avec ou sans agitation mécanique ou magnétique, et de préférence sous atmosphère contrôlée, par exemple sous atmosphère inerte d'argon. Dans les modes de réalisation de l'invention selon lesquels l'assemblage est formé en présence de CO₂, l'atmosphère contrôlée comprend de préférence du CO₂, en particulier il s'agit d'une atmosphère de CO₂. L'apport de CO₂ peut également être fait par bullage de CO₂ dans le milieu liquide comprenant les autres composants et l'échantillon susceptible de comprendre un élément chimique tel que défini ci-avant, par ajout d'un sel de carbonate et/ou d'hydrogénocarbonate, et/ou par ajout de carboglace audit milieu liquide.

Le milieu comprenant les différents composants et l'échantillon susceptible de comprendre un élément chimique tel que défini ci-avant est de préférence un milieu liquide.

La mise en contact des différents composants peut être réalisée soit séquentiellement, soit simultanément. De préférence, la mise en contact de l'amine, de l'aldéhyde et/ou l'imine et/ou le CO₂, ou de l'adduit formé par la mise en contact d'une amine et de CO₂ avec l'échantillon susceptible de comprendre l'élément chimique , est simultanée. Le terme « simultané » signifie que la mise en contact des différents composants est réalisée dans un laps de temps court, par exemple compris entre 0 et 30 minutes. En particulier, il est de l'ordre de moins de 15 minutes, moins de 10 minutes, moins de 5 minutes, moins de 2 minutes ou moins d'une minute. Dans un mode de réalisation préféré, l'ajout de l'échantillon susceptible de comprendre l'élément chimique est réalisé moins de 2 minutes après le mélange des autres composants.

L'ordre dans lequel les composants sont ajoutés peut être quelconque, et l'homme du métier est à même de déterminer l'ordre le plus approprié en fonction de la nature de chacun des composants. Dans un mode de réalisation préféré, l'échantillon susceptible de comprendre l'élément chimique est ajouté après les autres composants de l'assemblage.

La mise en contact des différents composants peut éventuellement se faire en présence d'autres composés, par exemple en présence d'au moins un solvant. Le solvant peut être choisi parmi n'importe quel solvant organique ou aqueux et adapté aux composants mis en contact, par exemple pour favoriser leur dissolution. Dans un mode de réalisation, le solvant est choisi parmi les alcools, par exemple il s'agit de l'éthanol ou du méthanol. Le solvant peut être utilisé sous forme anhydre ou à différents degrés d'hydratation.

Parmi les autres composés susceptibles d'être utilisés, on peut également citer les amines telles que définies ci-avant.

La formation de l'assemblage selon l'invention est visualisée par l'apparition d'un précipité, lequel peut être facilement séparé du milieu réactionnel, par exemple par une simple filtration et/ou centrifugation.

L'étape de détection et/ou de capture des éléments chimiques du procédé selon l'invention est donc réalisée par observation de ce précipité après la mise en contact des différents composants.

Avantageusement, l'étape de détection comprend en outre la comparaison de l'échantillon obtenu avec un échantillon similaire qui ne comprend pas l'élément chimique, et qui peut être nommé échantillon de référence.

De même, l'étape de détection peut comprendre en outre la comparaison de l'échantillon obtenu avec un échantillon similaire qui comprend l'élément chimique. En effet, la détection de la présence (ou l'absence) de l'élément à détecter et/ou capturer est validée par l'apparition d'un précipité dans le cas où l'élément est présent, et l'absence de précipité dans les mêmes conditions dans le cas où l'élément est absent.

Le procédé de détection et/ou capture selon l'invention peut être utilisé pour déterminer l'absence, la présence et/ou la quantité (absolue ou relative) d'au moins un élément chimique dans un échantillon.

Le procédé de détection et/ou capture selon l'invention comprend éventuellement une étape de séparation de l'assemblage moléculaire formé qui précipite.

L'étape de séparation peut être réalisée par toute technique adaptée connue de l'homme du métier. Par exemple, la séparation peut être effectuée par décantation, filtration, tamisage, centrifugation, évaporation et/ou distillation. Avantageusement, l'étape de séparation est réalisée par une simple filtration et/ou centrifugation. L'homme du métier est à même d'ajuster les paramètres de la technique choisie pour obtenir une séparation optimale. Par exemple, il est à même d'ajuster les paramètres de la filtration, tels que la nature du filtre et/ou la taille des pores, et/ou les paramètres de centrifugation, tels que la vitesse de centrifugation, à l'assemblage et à l'échantillon concernés, pour obtenir une séparation optimale.

L'échantillon sur lequel le procédé de détection et/ou capture selon l'invention est mis en oeuvre peut être n'importe quel type d'échantillon susceptible de comprendre un ou plusieurs éléments chimiques tels que définis ci-dessus. L'échantillon peut se présenter sous forme gazeuse, liquide ou solide, de préférence il se présente sous forme liquide ou solide. Dans le cas où l'échantillon susceptible de comprendre un ou plusieurs des éléments chimiques visés ne comprend effectivement aucun de ces éléments, ou en tout cas aucun des éléments permettant la formation d'un assemblage moléculaire avec les composants spécifiquement utilisés, le procédé de détection et/ou capture selon l'invention ne conduit pas à la formation d'un assemblage ni à l'observation d'un précipité. Dans un tel mode de réalisation, le procédé selon l'invention est un procédé de détection de l'absence dans l'échantillon d'au moins un des éléments chimiques visés. Cet échantillon peut être nommé échantillon de référence.

De préférence, le procédé de détection et/ou capture selon l'invention est réalisé au sein d'une bibliothèque combinatoire dynamique comprenant au moins une amine et au moins un aldéhyde et/ou imine et/ou du CO₂ ou un adduit formé par la mise en contact d'une amine et de CO₂ selon l'invention.

Le procédé de détection et/ou capture selon l'invention présente des avantages indéniables par rapport aux techniques de détection et/ou capture actuelles.

Ainsi, le procédé de capture selon l'invention n'implique pas nécessairement des températures élevées, et le procédé peut être avantageusement réalisé à température ambiante. Comparativement, les procédés métallurgiques sont généralement mis en oeuvre à des températures de plusieurs centaines de degrés.

Ensuite, les « briques moléculaires » qui constituent les assemblages, c'est-à-dire les aldéhydes, imines et/ou amines sont des composés simples, parfois commerciaux, qui ne nécessitent pas de synthèse à façon.

Ensuite, le procédé de séparation implique comme seule étape de séparation une simple séparation de phase, par exemple par filtration ou centrifugation, beaucoup plus simple et économique à mettre en oeuvre que d'autres techniques de séparation classiquement utilisées dans ce domaine, telles que les extractions ou les chromatographies, qui consomment en outre de grandes quantités de solvant.

Ensuite, le procédé selon l'invention peut permettre, notamment après relargage, le recyclage et/ou la réutilisation d'au moins certaines des « briques moléculaires » qui constituent les assemblages. Cette possibilité de réutilisation des composants permet de limiter, voire de supprimer, les effluents en sortie du procédé.

Enfin, les assemblages selon l'invention peuvent utiliser comme agent de capture le dioxyde de carbone, qui est un déchet.

Par conséquent, par rapport aux procédés de séparation classiquement utilisés pour les éléments chimiques envisagés dans la présente invention, le procédé selon la présente invention est clairement économique et peu énergivore, ce qui représente des atouts industriels majeurs.

En outre, il a été démontré que, dans certaines conditions où plusieurs éléments chimiques, de préférence plusieurs terres rares, sont présents, un assemblage selon l'invention peut se former sélectivement avec un seul type d'élément chimique. Par exemple, des facteurs de séparation pouvant monter jusqu'à 20 ont pu être obtenus pour la séparation de deux terres rares, comme par exemple La et Lu, dans un même échantillon en mettant en oeuvre un procédé selon l'invention.

Dans un mode de réalisation de l'invention, la détection et/ou capture d'au moins un élément chimique selon le procédé de l'invention est sélective d'un élément chimique.

Un autre objet de l'invention est un procédé de relargage de l'élément capté (ou des éléments captés) dans l'assemblage, par séparation ou dissociation, au moins partielle, de préférence totale, des composants de l'assemblage. Cette séparation peut être réalisée par exemple par chauffage, de préférence à une température inférieure ou égale à 100°C, ou par hydrolyse, en particulier par hydrolyse acide, basique ou en milieu tamponné neutre, au moins partielle, de l'assemblage. De préférence, la séparation des composants de l'assemblage permet de libérer et/ou reformer au moins certains des composants, de préférence tous les composants, de l'assemblage. Ainsi, dans un mode de réalisation, la séparation permet de libérer et/ou reformer au moins un composant, de préférence tous les composants, parmi les aldéhydes, les imines, les amines et/ou le CO₂ formant l'assemblage, ou un de leurs dérivés. L'expression « un de leurs dérivés » désigne un dérivé chimique de l'aldéhyde ou de l'amine, à partir duquel l'homme du métier sait reformer l'aldéhyde, ou l'amine. Par exemple, le procédé de relargage peut permettre de libérer un aldéhyde dans le cas où une imine avait été utilisée pour former l'assemblage. Une telle séparation permet de ré-utiliser les composants récupérés, par exemple dans un nouveau procédé selon l'invention, réduisant d'autant le coût de mise en oeuvre du procédé. Dans le cas où la dissociation de l'assemblage est réalisée par hydrolyse, le procédé de relargage peut comprendre en outre après l'hydrolyse une étape d'élimination de la solution aqueuse obtenue, afin de permettre la ré-utilisation des composants.

Dans le procédé de relargage selon l'invention, le ou les éléments chimiques choisis parmi les métaux pauvres, les alcalins, les alcalino-terreux, les actinides et les terres rares sont libérés soit sous forme d'élément chimique, soit sous forme d'un dérivé comprenant l'élément chimique, par exemple sous forme d'un complexe ou d'un sel.

Un autre objet de l'invention est un procédé de formation d'un assemblage moléculaire comprenant la mise en contact de :
- au moins une amine et au moins un aldéhyde et/ou une imine (formée par la mise en contact d'un aldéhyde et une amine) et/ou du CO₂, ou
- un adduit formé par la mise en contact d'une amine et de CO₂,
avec au moins un échantillon comprenant au moins un élément chimique choisi parmi les métaux pauvres, les alcalins, les alcalino-terreux, les actinides et les terres rares.

L'assemblage moléculaire formé par ce procédé est également un objet de l'invention.

Un autre objet de l'invention est un kit comprenant au moins une amine, et au moins un aldéhyde et/ou une imine et/ou du CO₂, ou un adduit formé par la mise en contact d'une amine et de CO₂, ces composants étant tels que définis précédemment.

Ce kit est adapté notamment pour la détection, la capture et/ou le relargage d'au moins un élément chimique selon l'invention. Le kit peut comprendre au moins deux aldéhydes et/ou imines et/ou au moins deux amines. Le kit peut notamment se présenter sous la forme d'un support adapté pour une utilisation en combinatoire dynamique, ledit support comprenant plusieurs puits, dans lesquels chaque puits comprend au moins une amine et éventuellement au moins un aldéhyde et/ou imine. Le support peut être par exemple une plaque comprenant des puits.

Sauf précision contraire, le terme « rendement » désigne dans la présente invention un rendement molaire.

Les avantages de l'invention ressortiront plus clairement des exemples ci-dessous, lesquels sont fournis uniquement à titre illustratif et non limitatif de l'invention.

### Exemples

### Exemple 1 : Préparation des composants de l'assemblage

### 1.1 Préparation des aldéhydes

Les aldéhydes B et C ont été synthétisés par triformylation de phénols mettant en jeu une réaction de Duff selon les conditions suivantes :

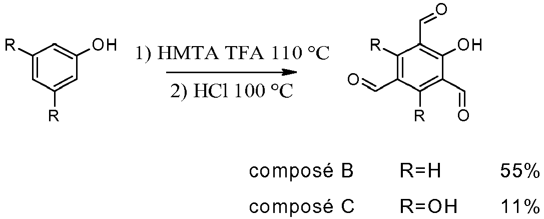

L'aldéhyde D a été synthétisé selon la réaction suivante : 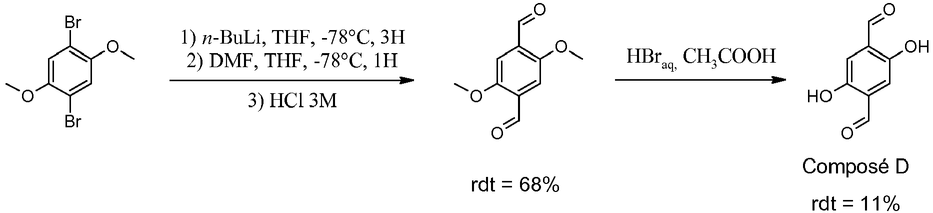 ou selon la réaction suivante :

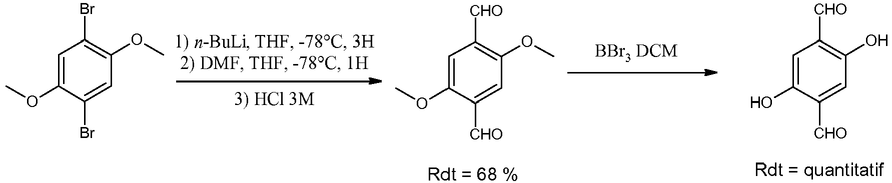

L'aldéhyde E a été synthétisé selon la réaction suivante :

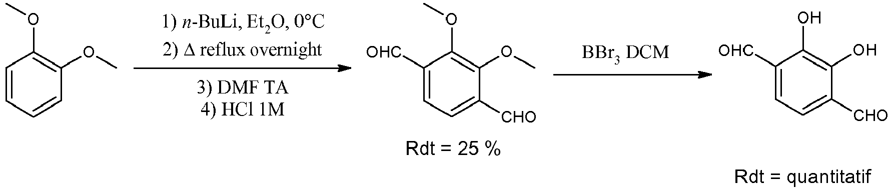

L'aldéhyde F a été synthétisé selon la réaction suivante :

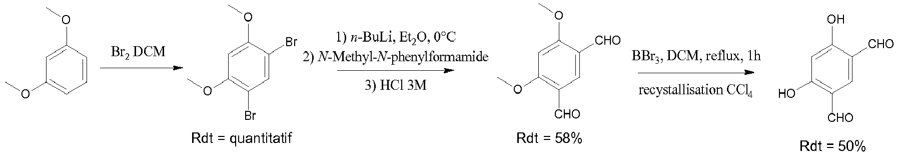

### 1.2 Préparation des imines

Les imines Al₃ et Bl₃ ont été formées par réaction des aldéhydes B et C avec la n-butylamine, selon la réaction suivante :

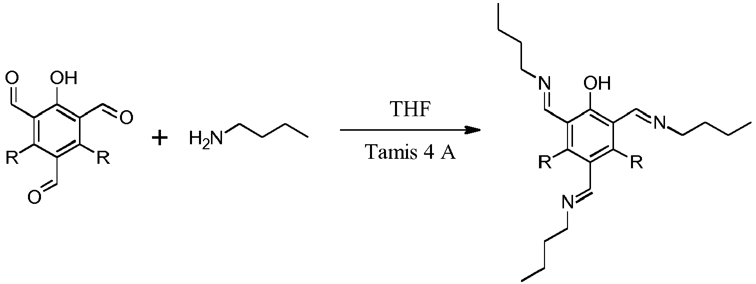

### 1.3 Préparation des sels de lanthanides

Les sels de lanthane, gadolinium et de lutécium ont été préparés à partir des oxydes chauffés à reflux pendant 12h dans un mélange d'acide trifluoroacétique et d'eau.

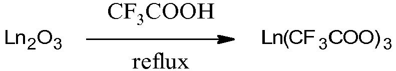

**La(CF₃COO)₃** : 2,99 g de La₂O₃ sont mis en suspension dans 32 mL d'eau distillée, ensuite 40 mL d'acide trifluoroacétique sont ajoutés goutte à goutte. Le mélange est agité à 110°C pendant une nuit. La solution est filtrée sur fritté, et le filtrat est évaporé à l'évaporateur rotatif. Le solide obtenu est repris trois fois à l'éthanol. Le sel (7g) est obtenu après séchage sous vide sous forme d'une poudre blanche avec un rendement de 90%.

**Lu(CF₃COO)₃:** 0,5 g de Lu₂O₃ sont mis en suspension dans 8 mL d'eau distillée, ensuite 10 mL d'acide trifluoroacétique sont ajoutés goutte à goutte. Le mélange est agité à 110°C pendant une nuit. La solution est filtrée sur fritté, et le filtrat est évaporé à l'évaporateur rotatif. Le solide obtenu est repris trois fois à l'éthanol. Le sel (1g) est obtenu après séchage sous vide sous forme d'une poudre blanche avec un rendement de 90%.

**Gd(CF₃COO)₃:** 5 g de Gd₂O₃ sont mis en suspension dans 80 mL d'eau distillée, ensuite 100 mL d'acide trifluoroacétique sont ajoutés goutte à goutte. Le mélange est agité à 110°C pendant une nuit. La solution est filtrée sur fritté, et le filtrat est évaporé à l'évaporateur rotatif. Le solide obtenu est repris trois fois à l'éthanol. Le sel (11g) est obtenu après séchage sous vide sous forme d'une poudre blanche avec un rendement de 90%.

### 1.4 Préparation des nitrates d'indium, de néodyme, de praséodyme, et de lutétium

Les nitrates d'indium In(NO₃)₃, de néodyme Nd(NO₃)₃ de praséodyme Pr(NO₃)₃ et de lutétium Lu(NO₃)₃ ont été obtenus respectivement par attaque de l'oxyde d'indium, du néodyme, de praséodyme et de lutétium avec de l'acide nitrique. 1 g d'oxyde correspondant est dispersé dans 20 ml d'eau distillée, 10 ml d'acide nitrique à 68 % sont ajoutés. Le mélange est chauffé à reflux jusqu'à dissolution complète de l'oxyde, et après retour à température ambiante la solution est filtrée.

Le sel de nitrate est obtenu après évaporation complète du solvant et séchage à la pompe à palette.

### 1. 5 Préparation des acétates d'indium, dde néodyme, de praséodyme, et de lutétium

Les nitrates d'indium In(CH₃CO₂)₃, de néodyme Nd(CH₃CO₂)₃, de praséodyme Pr(CH₃CO₂)₃ et de lutétium Lu(CH₃CO₂)₃ ont été obtenus respectivement par attaque de l'oxyde d'indium, de néodyme, de praséodyme et de lutétium avec de l'acide acétique. 1 g d'oxyde correspondant est dispersé dans 20 ml d'eau distillée, 20 ml d'acide nitrique à 68 % sont ajoutés. Le mélange est chauffé à reflux jusqu'à dissolution complète de l'oxyde, et après retour à température ambiante la solution est filtrée. Le sel de nitrate est obtenu après évaporation complète du solvant et séchage à la pompe à palette.

### Exemple 2 : Mise en évidence de la formation des assemblages par chimie combinatoire dynamique

Les différentes bibliothèques ont été préparées par mélange d'un équivalent d'aldéhyde (ou imine), un équivalent de sel métallique, et deux équivalents d'amine. La concentration finale dans les bibliothèques est de 20mM en aldéhyde ou imine, 20 mM en sel métallique et 40mM en amine.

Les espèces utilisées pour les bibliothèques ont été abrégées selon les numéros de composés fournis dans la description. Dans le cas de bibliothèques préparées à partir d'aldéhydes, un équivalent de n-butylamine par fonction aldéhyde a été introduit afin d'obtenir des mélanges initialement solubles.

Les bibliothèques ont été placées sous atmosphère de CO₂ ou d'argon. Les bibliothèques ont été dupliquées trois fois.

A titre d'exemples des résultats obtenus, les figures 1 et 2 regroupent l'analyse des bibliothèques pour chacune des conditions testées, pour les deux temps d'expérience 15 minutes et 2880 minutes. Les cases grisées dans les tableaux correspondent aux expériences pour lesquelles un précipité (caractéristique de la formation d'un assemblage selon l'invention) a pu être observé.

Il est clair que la mise en contact, selon l'invention, d'un aldéhyde ou d'une imine, d'une amine, éventuellement de dioxyde de carbone, et d'un élément chimique choisi parmi le lanthane et le lutétium a permis la formation d'un assemblage selon l'invention dans un grand nombre de conditions.

Des expériences ont également été réalisées en supprimant des milieux réactionnels soit l'aldéhyde (ou l'imine), soit l'amine, soit l'élément chimique choisi parmi le lanthane et le lutétium, et dans chaque cas la formation d'un précipité caractéristique de la formation d'un assemblage selon l'invention n'a pas pu être observée. Dans certains cas, aucune précipitation n'a eu lieu, et dans d'autres cas seul un précipité fin et peu abondant qui ne peut être isolé a été formé.

### Exemple 3 : Tests de sélectivité

Les bibliothèques ont été préparées par mélange d'un équivalent d'aldéhyde (ou imine), un équivalent de sel de lanthane, un équivalent de sel de lutécium et deux équivalents d'amine. La concentration finale dans les bibliothèques est de 20mM en aldéhyde (ou imine), 20 mM en lanthane, 20 mM en lutécium et 40mM en amine. Le volume final de la bibliothèque est de 6 mL. Les précipités ont été séparés dès l'observation de la présence d'un précipité. Les résultats de l'analyse élémentaire des précipités obtenus sont regroupés dans le tableau 1 ci-dessous.

**Tableau 1 : Analyse élémentaire des précipités obtenus**

| | Précipité | Analyse élémentaire | | | | | |
|---|---|---|---|---|---|---|---|
| | | C | H | La | Lu | N | Rapport La/Lu |
| Ar | A : 3 : La/Lu | 28,19 | 3,45 | 12,25 | 17,41 | 6,79 | 0,70 |
| | A : 4 : La/Lu | 29,78 | 3,83 | 12,01 | 16,32 | 6,32 | 0,74 |
| | Al₃ : 3 : La/Lu | 29,39 | 3,60 | 11,16 | 15,97 | 6,94 | 0,70 |
| | C : 3 : La/Lu | 31,30 | 3,87 | 10,16 | 15,09 | 6,87 | 0,67 |
| | C : 4 : La/Lu | 28,20 | 3,43 | 10,96 | 15,09 | 5,04 | 0,66 |
| | C : 5 : La/Lu | 28,85 | 3,41 | 10,38 | 19,09 | 4,26 | 0,54 |
| | B1₃ : 3 : La/Lu | 31,07 | 3,91 | 10,73 | 14,34 | 6,99 | 0,75 |
| | | | | | | | |
| CO₂ | A : 4 : La/Lu | 32,51 | 4,14 | 10,91 | 12,61 | 8,37 | 0,87 |
| | A : 5 : La/Lu | 29,79 | 3,82 | 12,75 | 14,87 | 6,75 | 0,86 |
| | A : 6 : La/Lu | 28,62 | 3,61 | 12,27 | 13,61 | 8,95 | 0,90 |
| | C : 2 : La/Lu | 37,60 | 3,94 | 2,98 | 20,22 | 4,94 | 0,15 |
| | C : 5 : La/Lu | 35,40 | 3,94 | 7,00 | 18,62 | 5,02 | 0,38 |

L'analyse élémentaire confirme la présence de lanthanides dans les précipités, donc une capture des ions métalliques lors de la formation des précipités. De plus, l'analyse élémentaire montre (voir colonne rapport La/Lu) que certaines conditions, en particulier les solides préparés en présence de CO₂ et de l'aldéhyde C, présentent une sélectivité intéressante vis-à-vis du Lutétium.

La reproduction des conditions de la dernière ligne du tableau (C : 5 : La/Lu en présence de CO₂) dans une manipulation isolée (c'est-à-dire pas dans une bibliothèque) a permis d'augmenter la sélectivité La/Lu jusqu'à 2,52.

Le mode opératoire mis en oeuvre est le suivant :
12,6 mg de C (6 10⁻⁵ mol) sont placés dans 3 ml de méthanol, 12 µl de butylamine (1,2 10⁻⁴ mol) sont ajoutés pour solubiliser le tris-aldéhyde. 17,43 mg de spermidine 5 (1,2 10⁻⁴ mol), 30,8 mg de Lu(CF₃CO₂) (3 10⁻⁵ mol) et 28,7 mg de La(CF₃CO₂) (3 10⁻⁵ mol) sont ajoutés à la solution. Du CO₂ est bullé dans la solution jusqu'à l'apparition du précipité. Le solide est centrifugé, lavé trois fois au méthanol puis séché sous flux de CO₂.

| Ln introduit | | Contre anion | Stoechiométrie en Ln | | Mesure ICP-MS % massique | | % rapport des masses engagées¹ | | Ln1/ Ln2 |
|---|---|---|---|---|---|---|---|---|---|
| | | | | | **La** | **Lu** | **La** | **Lu** | |
| La | Lu | CF₃CO₂⁻ | 0,5 | 0,5 | 24,73 | 9,82 | 6,35 | 8,00 | 2,52 |

1) Rapport des masses engagées = m_{M}⁰/(m₅⁰+m_{B}⁰+m_{NHB}u⁰+m_{CO2}^{max}+m_{M}⁰) avec m_{M}⁰ la masse de métal introduite, m₅⁰ la masse de la spermidine introduite, m_{C}⁰ la masse d'aldéhyde introduite, m_{NHB}u⁰ la masse de butylamine introduite et m_{CO2}^{max} la masse maximale de CO₂ pouvant être capté.

Le terme ICP-MS désigne la spectrométrie de masse par torche à plasma.

### ➢ Analyse de l'influence de la concentration en lanthanide sur la sélectivité

Des solides obtenus en présence de 1 et 0,5 équivalent de Lanthane et de Lutétium ont été analysés par LC-MS. Les bibliothèques avec 1 équivalent de La et Lu ont été préparées en mélangeant 1 mL d'aldéhyde à 60 mM, 1 mL d'amine à 120 mM, 500µL de La(CF₃COO)₃ à 120 mM et 500µL de Lu(CF₃COO)₃ à 120 mM. Les bibliothèques avec 0,5 équivalent de La et Lu ont été préparées en mélangeant 1 mL d'aldéhyde à 60 mM, 1 mL d'amine à 120 mM, 250µL de La(CF₃COO)₃ à 120 mM, 250µL de Lu(CF₃COO)₃ à 120 mM et 500 µL de méthanol. Le dosage des lanthanides effectué en LC-MS est présenté dans le tableau 2 ci-dessous.

**Tableau 2 : Influence de la concentration en métal dans les bibliothèques sur la sélectivité**

| Précipité | Nombre d'équivalents en métal | Quantité (mg) | % massique | | % de métal capté | | Rapport massique La/Lu |
|---|---|---|---|---|---|---|---|
| | | | La | Lu | La | Lu | |
| A : 5 : CO₂ | ½ | 30 | 4,99 | 7,44 | 35,9 | 42,5 | 0,67 |
| A : 5 :CO₂ | 1 | 4 | 4,39 | 9,16 | 2,1 | 3,5 | 0,48 |
| C : 2 : CO₂ | ½ | 7 | 0,14 | 3,29 | 0,2 | 4,4 | 0,04 |
| C : 2 : CO₂ | 1 | 13,1 | 2,45 | 8,90 | 3,8 | 11,11 | 0,27 |
| C : 4 : CO₂ | ½ | 4,7 | < seuil | 3,18 | / | 2,8 | / |
| C : 4 : CO₂ | 1 | 16,4 | 2,33 | 12,24 | 4,6 | 19,1 | 0,19 |
| C : 5 : CO₂ | ½ | 10 | 0,87 | 9,15 | 2,1 | 17,4 | 0,09 |
| C : 5 : CO₂ | 1 | 10,7 | 2,30 | 9,94 | 3,0 | 10,1 | 0,23 |
| C : 6 : CO₂ | 1 | 12,4 | 0,56 | 6,98 | 0,8 | 8,2 | 0,08 |

Ces résultats montrent notamment une bonne sélectivité des bibliothèques préparées à partir de l'aldéhyde C. En outre, la diminution de la concentration en métal dans les bibliothèques entraîne une augmentation de la sélectivité lors de la formation des précipités.

### Exemple 4 : Capture de différents éléments avec la poléthylèneimine

### Modes opératoires :

12 mg (m_{In}⁰, 4 10⁻⁵ mol) d'In(NO₃)₃ sont dissous dans 6 ml d'éthanol absolu, 0,5 ml de PEI (polyéthylèneimine, Mw=60 000g/mol) à 50 % dans l'eau (m_{PEI}⁰ = 250 mg, n_{PEI}⁰ = 4,1 10⁻⁶ mol ; 5,72 10⁻³ mol de N ; n_{CO2}^{max} = 2,86.10⁻³ ; n_{In}^{max} = 9.53 10⁻⁴ mol) sont ajoutés (solution limpide). Du CO₂ est bullé dans la solution à température et pression atmosphériques. On observe la formation immédiate d'un précipité. La suspension est chauffée à 90°C sous pression de CO₂ jusqu'à dissolution complète du solide. La solution est alors ramenée lentement à température ambiante. Le solide est obtenu par simple filtration, lavage et séchage sous vide.

9,1 mg (m_{Pr}⁰, 2,1 10⁻⁵ mol) de Pr(NO₃)₃ et 10,3 mg (ni_{Lu}⁰, 2,8 10⁻⁵ mol) de Lu(NO₃)₃ sont dissous dans 6 ml d'éthanol absolu, 0,5 ml de PEI (polyéthylèneimine, Mw=60 000g/mol) à 50 % dans l'eau (m_{PEI}⁰ = 250 mg, n_{PEI}⁰ = 4,1 10⁻⁶ mol; 5,72 10⁻³ mol de N ; n_{CO2}^{max} = 2,86.10⁻³) sont ajoutés (solution limpide). Du CO₂ est bullé dans la solution à température et pression atmosphériques. On observe la formation immédiate d'un précipité. La suspension est chauffée à 90°C sous pression de CO₂ jusqu'à dissolution complète du solide. La solution est alors ramenée lentement à température ambiante ce qui provoque la formation d'un nouveau solide. Le solide est obtenu par simple filtration, lavage et séchage sous vide.

### Résultats :

| T °C | élément introduit | | Ln1/Ln2 | Mesure ICP-MS % massique | | | Rapport des masses engagées¹ | | | % massique maximal théorique² | | | % de métal capté |
|---|---|---|---|---|---|---|---|---|---|---|---|---|---|
| | | | | **In** | **Pr** | **Lu** | **In** | **Pr** | **Lu** | **In** | **Pr** | **Lu** | **In** |
| 90 | In | | | 1,02 | | | 1,18 | | | 22,54 | | | 100 |
| 25 | In | | | 0,78 | | | 1,18 | | | 22,54 | | | 95 |
| 90 | Pr | Lu | 0,75 | | 0,74 | 0,98 | | 0,73 | 0,91 | | 12,57 | 15,61 | |

1) Rapport des masses engagées = m_{M}⁰/(m_{PEI}⁰+m_{M}⁰+m_{CO2}^{max}) avec m_{M}⁰ la masse de métal introduite et m_{PEI}⁰ la masse de PEI introduite.
2) % massique maximal théorique = m_{M}^{max}/(m_{PEI}⁰+m_{M}^{max}+m_{co2}^{max}) avec m_{M}^{max} la masse maximale de métal pouvant être capté et m_{CO2}^{max} la masse maximale de CO₂ pouvant être capté, pour une masse de PEI égale à m_{PEI}⁰.

La PEI a permis la capture d'In, Pr et Lu. Les teneurs en métaux dans les solides analysés sont faibles (inférieures à 1 %). Cependant, elles sont du même ordre de grandeur que le ratio masse de PEI sur masse de métal utilisé pour la capture et sont cohérentes avec l'augmentation de masse du polymère par absorption de CO₂. Dans le cas de l'indium, les solides obtenus par précipitation lente (descente en température à partir de 90 °C) ont une plus forte teneur en métal que les solides obtenus par capture à température ambiante. La sélectivité entre Pr et Lu est en faveur du Lu avec un rapport Pr/Lu de 0,75.

### Exemple 5 : Capture de différents éléments avec des amines de faible masse moléculaire

Les amines suivantes sont utilisées :
TETA :Triéthylènetétramine : 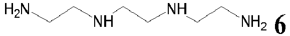
DETA : Diéthylènetriamine : 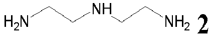
En : Ethylènediamine : 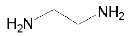
Tren: tris-(2-aminoéthyl)amine :

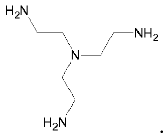

### Mode opératoire :

A une solution d'amine (n_{A}= 6 10⁻⁴ mol) dans 5 ml d'éthanol absolu est ajoutée une quantité de métal comprise entre 0,13 et 0,04. Du CO₂ est bullé dans la solution à température et pression atmosphériques. La suspension est chauffée à 90°C sous pression de CO₂ jusqu'à dissolution complète du solide. La solution est alors ramenée lentement à température ambiante (1 à 2°C/min) afin de provoquer la précipitation. Le solide est obtenu par filtration, lavage à l'éthanol absolu et séchage sous vide.

### Résultats :

La réversibilité des solides a été vérifiée lors du chauffage de la suspension à 90 °C. Les solides formés lors de la descente lente en température ont été analysés par ICP-MS (cf tableau ci-dessous).

| T °C | Amine utilisée | Elément introduit | Contre ion | n_{A}/n_{M} | Mesure ICP-MS % massique | | Rapport des masses engagées¹ en % | | % massique maximal théorique² | | % de métal capté |
|---|---|---|---|---|---|---|---|---|---|---|---|
| | | | | | **In** | **Nd** | **In** | **Nd** | **In** | **Nd** | **In** |
| 90 | TETA | In | NO₃⁻ | 6 | 3,45 | | 7,55 | | 24,63 | | 53 |
| 90 | TETA | Nd | NO₃⁻ | 24 | | 2,36 | | 3,30 | | 29,10 | |
| 90 | TETA | Nd | ⁻OAc | 12 | | 6,10 | | 4,88 | | 29,10 | |
| 90 | TETA | Nd | CF₃CO₂⁻ | 6 | | 14,25 | | 9,31 | | 29,10 | |
| 90 | Tren | In | NO₃⁻ | 6 | 2,38 | | 8,27 | | 21,29 | | 20 |
| 90 | DETA | In | NO₃⁻ | 6 | 6,55 | | 10,16 | | 25,34 | | 56 |
| 90 | En | In | NO₃⁻ | 24 | 5,01 | | 4,39 | | 26,88 | | 58 |
| 25 | TETA | In | NO₃⁻ | 6 | 1,52 | | 7,55 | | 24,63 | | 88 |
| 25 | TETA | Nd | NO₃⁻ | 24 | | 2,02 | | 3,30 | | 29,10 | |
| 25 | TETA | Nd | ⁻OAc | 12 | | 3,06 | | 4,88 | | 29,10 | |
| 25 | TETA | Nd | CF₃CO₂⁻ | 6 | | 6,64 | | 9,31 | | 29,10 | |
| 25 | Tren | In | NO₃⁻ | 6 | 12,35 | | 8,27 | | 21,29 | | 51 |
| 25 | DETA | In | NO₃⁻ | 6 | 2,98 | | 10,16 | | 25,34 | | 71 |
| 25 | En | In | NO₃⁻ | 24 | 3,49 | | 4,39 | | 26,88 | | 100 |

1) Rapport des masses engagées = m_{M}⁰/(m_{A}⁰+m_{CO2}^{max}+m_{M}⁰) avec m_{M}⁰ la masse de métal introduite, m_{A}⁰ la masse d'amine introduite et m_{CO2}^{max} la masse maximale de CO₂ pouvant être capté avec une masse m_{A}⁰ d'amine engagée.
2) % massique maximal théorique = m_{M}^{max}/(m_{A}⁰+m_{M}^{max}+m_{co2}^{max}) avec m_{M}^{max} la masse maximale de Métal pouvant être capté et m_{CO2}^{max} la masse maximale de CO₂ pouvant être capté avec une masse m_{A}⁰ d'amine engagée.

Les carbamates d'amines ayant deux à quatre sites azotes ont permis la capture de l'In et du Nd, les teneurs en métaux varient entre 2 et 15 % suivant le métal, le contre ion et l'amine utilisés.

Dans le cas de Nd, la teneur en métal augmente lorsque le ratio métal/amine initialement introduit augmente, ce qui signifie que les stoechiométries utilisées sont sous-saturantes. Les valeurs des teneurs sont proches des rapports des masses engagées, dans certains cas elles sont même supérieures à ces rapports. On peut en déduire dans ces cas, on assiste à un phénomène de déplacement d'équilibre et qu'une partie de l'amine est restée en solution sous forme libre. On observe également un rôle du contre-ion du métal sur la réversibilité de la capture selon le régime (cinétique/thermodynamique).

Dans le cas de l'In, la capture est plus efficace à 90 °C dans le cas des amines linéaires (En, DETA, TETA) et est plus efficace à TA dans le cas de la Tren.

### Exemple 6 : Etude de la capture d'In par le système A2CO₂

206,1 mg de trisimine Al₃ (6 10⁻⁴ mol) ont été dissous dans 10 ml de Méthanol, 129,6 µl de DETA (1,2 10⁻³ mol) et 54,76 mg d'In(NO₃)₃ (1,8 10⁻⁴ mol) ont été ajoutés à la solution. Du CO₂ a été bullé dans la solution jusqu'à l'apparition d'un précipité. La suspension a été chauffée à reflux sous atmosphère de CO₂ jusqu'à dissolution complète du solide. La solution a été refroidie lentement jusqu'à température ambiante. Le solide formé a été filtré, lavé au méthanol et séché sous vide.

| T°C | Ln | Mesure ICP-MS % massique | rapport des masses engagées | % massique maximal théorique |
|---|---|---|---|---|
| 90 | In | 2,42% | 7,58% | 19,75% |

La mesure d'ICP-MS montre que ce système permet la capture réversible de l'In à partir d'une solution dans l'alcool.

## Revendications

1. Procédé de détection et/ou capture d'au moins un élément chimique choisi parmi les métaux pauvres, les alcalins, les alcalino-terreux, les actinides et les terres rares dans un échantillon, comprenant la mise en contact de :
- au moins une amine et au moins un aldéhyde et/ou une imine et/ou du CO₂, ou
- un adduit formé par la mise en contact d'une amine et de CO₂,
avec un échantillon susceptible de comprendre au moins un desdits éléments chimiques.

2. Procédé selon la revendication 1, dans lequel au moins un des éléments chimiques est une (ou des) terre(s) rare(s).

3. Procédé selon la revendication 2, dans lequel les terres rares sont choisies parmi le lanthane et le lutétium.

4. Procédé selon l'une quelconque des revendications 1 à 3, dans lequel l'aldéhyde répond à la formule (III) 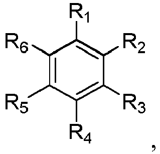 dans laquelle au moins un parmi R₁ à R₆ est une fonction aldéhyde, et les autres sont choisis parmi des atomes d'hydrogène, des hydroxyles, des thiols, des atomes d'halogène, des alkoxyles en C₁-C₂₀ et des alkyles en C₁-C₂₀.

5. Procédé selon l'une quelconque des revendications 1 à 4, dans lequel l'aldéhyde est choisi parmi les composés :

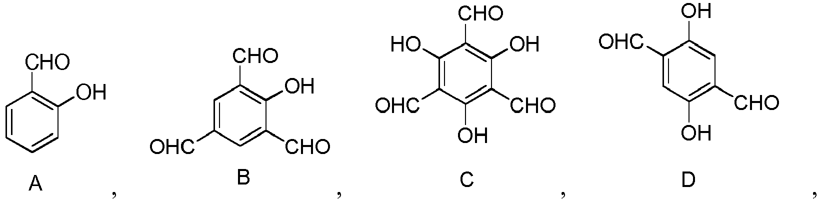

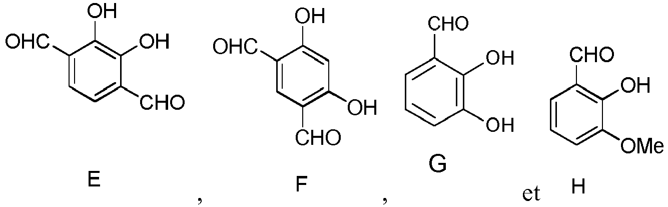

6. Procédé selon l'une quelconque des revendications 1 à 5, dans lequel l'amine est de formule générale (IV) R2-NH-R3, dans laquelle :
- R2 est choisi parmi les groupes alkyles, alcényles, alcynyles, cycloalkyles, cycloalcényles, cycloalcynyles et les groupes aromatiques, dont la chaîne hydrocarbonée est éventuellement interrompue par au moins un hétéroatome choisi parmi N, O et S, et éventuellement substitués par au moins un substituant, et
- R3 est choisi parmi un atome d'hydrogène et les groupes alkyles, alcényles, acynyles, cycloalkyles, cycloalcényles, cycloalcynyles et les groupes aromatiques, dont la chaîne hydrocarbonée est éventuellement interrompue par au moins un hétéroatome choisi parmi N, O et S, et éventuellement substitués par au moins un substituant, lequel substituant ne comprend pas d'aldéhyde CHO.

7. Procédé selon la revendication 6, dans lequel R2 est un groupe alkyle linéaire dont la chaîne hydrocarbonée est éventuellement interrompue par au moins un hétéroatome choisi parmi N, O et S, et éventuellement substitué par au moins un substituant, et R3 est un atome d'hydrogène.

8. Procédé selon l'une des revendications 1 à 7, dans lequel l'amine est choisie parmi les composés 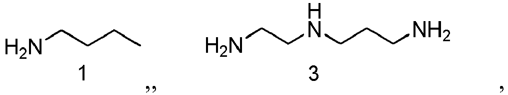 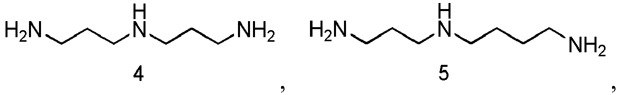 l'éthylènediamine 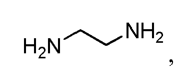 la tris-(2-aminoéthyl)amine 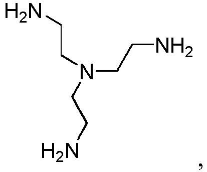 la diéthylènetétramine 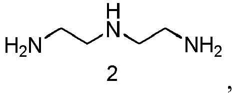 la triéthylènetétramine 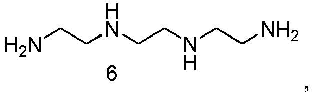 la pipérazine 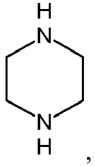 la cis-1,4-cyclohexanediamine 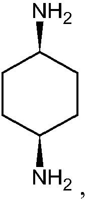 la trans-1,4-cyclohexanediamine 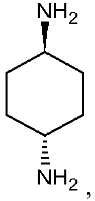 et la phénylènediamine

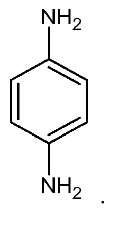

9. Procédé selon l'une quelconque des revendications 1 à 8, comprenant la mise en contact d'au moins une amine, et de CO₂, ou d'un adduit formé par la mise en contact d'une amine et de CO₂, avec au moins un desdits éléments chimiques.

10. Procédé selon l'une quelconque des revendications 1 à 9, dans lequel le procédé est mis en oeuvre dans des conditions de chimie combinatoire dynamique.

11. Procédé selon l'une quelconque des revendications 1 à 10, comprenant en outre une étape d'observation de la formation d'un précipité.

12. Procédé de relargage d'au moins un élément chimique choisi parmi les métaux pauvres, les alcalins, les alcalino-terreux, les actinides et les terres rares comprenant la séparation, au moins partielle, de préférence totale, des composants d'un assemblage formé par la mise en contact de :
- au moins une amine et au moins un aldéhyde et/ou une imine (formée par la mise en contact d'un aldéhyde et une amine) et/ou du CO₂, ou
- un adduit formé par la mise en contact d'une amine et de CO₂,
avec un échantillon comprenant au moins un desdits éléments chimiques.

13. Procédé de relargage d'au moins un élément chimique selon la revendication 12, dans lequel la séparation des composants est réalisée par chauffage et/ou par hydrolyse.

14. Procédé de relargage d'au moins un élément chimique selon la revendication 12 ou 13, dans lequel la séparation permet de libérer et/ou reformer au moins un composant, de préférence tous les composants, parmi les aldéhydes, les imines, les amines et/ou le CO₂ formant l'assemblage, ou un de leurs dérivés.

15. Kit pour la détection, la capture et/ou le relargage d'au moins un élément chimique choisi parmi les métaux pauvres, les alcalins, les alcalino-terreux, les actinides et les terres rares, comprenant au moins une amine, et au moins un aldéhyde et/ou une imine et/ou du CO₂, ou un adduit formé par la mise en contact d'une amine et de CO₂, dans lequel le kit se présente sous la forme d'un support adapté pour une utilisation en chimie combinatoire dynamique et dans lequel l'amine et l'aldéhyde sont tels que définis dans l'une des revendications 1 à 8.

## Patentansprüche

1. Verfahren zur Erkennung und/oder Aufnahme wenigstens eines chemischen Elements, ausgewählt aus p-Block-Metallen, Alkalimetallen, Erdalkalimetallen, Actinoiden und Seltenerdmetallen in einer Probe, umfassend Inkontaktbringen:
- wenigstens eines Amins und wenigstens eines Aldehyds und/oder eines Imins und/oder CO₂, oder
- eines Addukts, gebildet durch Inkontaktbringen eines Amins und CO₂,
mit einer Probe, die wenigstens eines der besagten chemischen Elemente umfassen kann.

2. Verfahren gemäß Anspruch 1, wobei wenigstens eines der chemischen Elemente ein Seltenerdmetall (oder Seltenerdmetalle) ist.

3. Verfahren gemäß Anspruch 2, wobei die Seltenerdmetalle aus Lanthan und Lutetium ausgewählt sind.

4. Verfahren gemäß einem der Ansprüche 1 bis 3, wobei der Aldehyd der Formel (III) 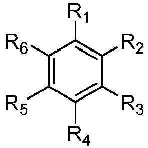 entspricht, in der wenigstens ein Rest von R1 bis R6 eine Aldehyd-Funktion ist und die anderen Reste aus Wasserstoffatomen, Hydroxylen, Thiolen, Halogenatomen, C₁-C₂₀-Alkoxylen und C₁-C₂₀-Alkylen ausgewählt sind.

5. Verfahren gemäß einem der Ansprüche 1 bis 4, wobei der Aldehyd aus den Verbindungen ausgewählt ist:

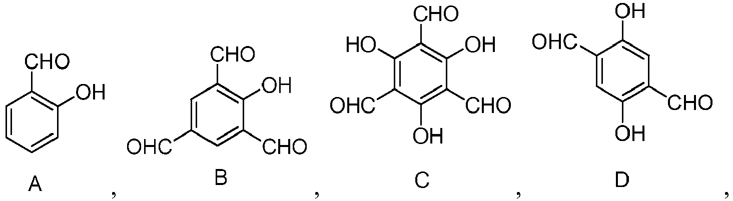

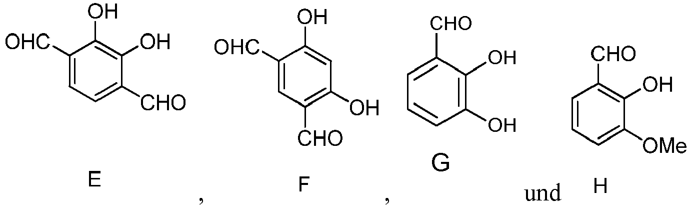

6. Verfahren gemäß einem der Ansprüche 1 bis 5, wobei das Amin die allgemeine Formel (IV) R2-NH-R3 besitzt, in der
- R2 aus Alkyl-, Alkenyl-, Alkinyl-, Cycloalkyl-, Cycloalkenyl-, Cycloalkinyl-Gruppen und aromatischen Gruppen ausgewählt ist, deren Kohlenwasserstoffkette eventuell durch wenigstens ein Heteroatom unterbrochen ist, das aus N, O und S ausgewählt ist, und eventuell mit wenigstens einem Substituenten substituiert sind, und
- R3 aus einem Wasserstoffatom und Alkyl-, Alkenyl-, Alkinyl-, Cycloalkyl-, Cycloalkenyl-, Cycloalkinyl-Gruppen und aromatischen Gruppen ausgewählt ist, deren Kohlenwasserstoffkette eventuell durch wenigstens ein Heteroatom unterbrochen ist, das aus N, O und S ausgewählt ist, und eventuell mit wenigstens einem Substituenten substituiert sind, wobei der Substituent keinen Aldehyd CHO umfasst.

7. Verfahren gemäß Anspruch 6, wobei R2 eine lineare Alkyl-Gruppe ist, deren Kohlenwasserstoffkette eventuell durch wenigstens ein Heteroatom unterbrochen ist, das aus N, O und S ausgewählt ist, und eventuell mit wenigstens einem Substituenten substituiert ist, und R3 ein Wasserstoffatom ist.

8. Verfahren gemäß einem der Ansprüche 1 bis 7, wobei das Amin aus den Verbindungen ausgewählt ist: 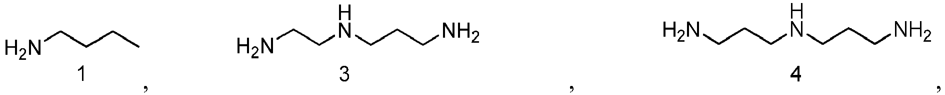 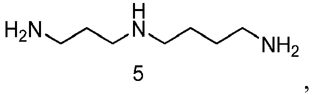 Ethylendiamin 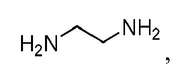 Tris-(2-aminoethyl)amin 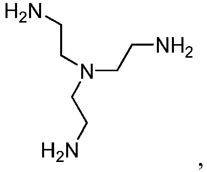 Diethylentetramin 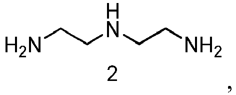 Triethyltetramin 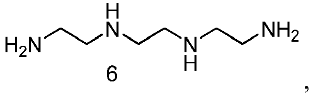 Piperazin 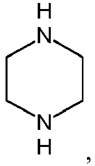 cis-1,4-Cyclohexandiamin 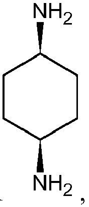 trans-1,4-Cyclohexandiamin 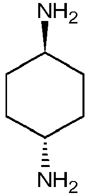 und Phenylendiamin

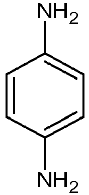

9. Verfahren gemäß einem der Ansprüche 1 bis 8, umfassend Inkontaktbringen wenigstens eines Amins und CO₂ oder eines Addukts, gebildet durch Inkontaktbringen eines Amins und CO₂, mit wenigstens einem der besagten chemischen Elemente.

10. Verfahren gemäß einem der Ansprüche 1 bis 9, wobei das Verfahren unter Bedingungen der dynamischen kombinatorischen Chemie durchgeführt wird.

11. Verfahren gemäß einem der Ansprüche 1 bis 10, umfassend außerdem einen Beobachtungsschritt der Bildung eines Niederschlags.

12. Verfahren zur Freisetzung wenigstens eines chemischen Elements, ausgewählt aus p-Block-Metallen, Alkalimetallen, Erdalkalimetallen, Actinoiden und Seltenerdmetallen, umfassend wenigstens die partielle, vorzugsweise die vollständige, Trennung der Komponenten eines Aufbaus, gebildet durch Inkontaktbringen:
- wenigstens eines Amins und wenigstens eines Aldehyds und/oder eines Imins (gebildet durch Inkontaktbringen eines Aldehyds und eines Amins) und/oder CO₂, oder
- eines Addukts, gebildet durch Inkontaktbringen eines Amins und CO₂, mit einer Probe, umfassend wenigstens eines der besagten chemischen Elemente.

13. Verfahren zur Freisetzung wenigstens eines chemischen Elements gemäß Anspruch 12, wobei die Trennung der Komponenten durch Erhitzen und/oder durch Hydrolyse erfolgt.

14. Verfahren zur Freisetzung wenigstens eines chemischen Elements gemäß Anspruch 12 oder 13, wobei die Trennung die Freisetzung und/oder Reformierung wenigstens einer Komponente, vorzugsweise aller Komponenten, von den Aldehyden, den Iminen, den Aminen und/oder CO₂, die den Aufbau bilden, oder einem ihrer Derivate gestattet.

15. Kit für die Erkennung, die Aufnahme und/oder die Freisetzung wenigstens eines chemischen Elements, ausgewählt aus p-Block-Metallen, Alkalimetallen, Erdalkalimetallen, Actinoiden und Seltenerdmetallen, umfassend wenigstens ein Amin und wenigstens einen Aldehyd und/oder ein Imin und/oder CO₂ oder ein Addukt, gebildet durch Inkontaktbringen eines Amins und CO₂, wobei das Kit in Form eines Trägers vorliegt, der für eine Verwendung in der dynamischen kombinatorischen Chemie geeignet ist, und wobei das Amin und der Aldehyd wie in einem der Ansprüche 1 bis 8 definiert vorliegen.

## Claims

1. A process for detecting and/or capturing at least one chemical element chosen from poor metals, alkali metals, alkaline earth metals, actinide metals and rare earth metals in a sample which comprises bringing:
- at least one amine and at least one aldehyde and/or one imine and/or CO₂, or
- an adduct formed by bringing an amine and CO₂ into contact,
into contact with a sample that may comprise at least one of said chemical elements.

2. The process as claimed in claim 1, in which at least one of the chemical elements is (a) rare earth metal(s).

3. The process as claimed in claim 2, in which the rare earth metals are chosen from lanthanum and lutetium.

4. The process as claimed in any one of claims 1 to 3, in which the aldehyde corresponds to the formula (III) 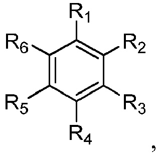 in which at least one from R₁ to R₆ is an aldehyde functional group and the others are chosen from hydrogen atoms, hydroxyls, thiols, halogen atoms, C₁-C₂₀ alkoxyls and C₁-C₂₀ alkyls.

5. The process as claimed in any one of claims 1 to 4, in which the aldehyde is chosen from the compounds:

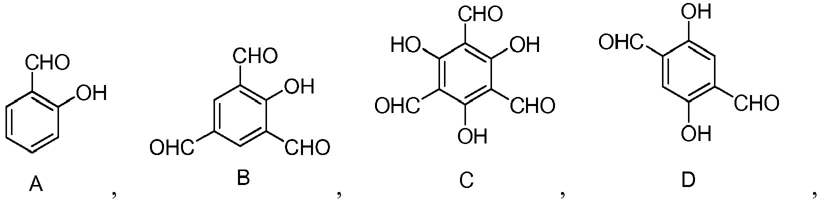

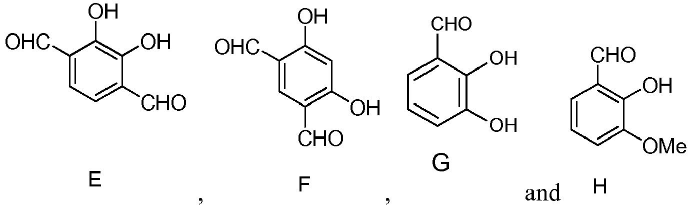

6. The process as claimed in any one of claims 1 to 5, in which the amine is of general formula (IV) R2-NH-R3, in which:
- R2 is chosen from alkyl, alkenyl, alkynyl, cycloalkyl, cycloalkenyl and cycloalkynyl groups and aromatic groups, the hydrocarbon chain of which is optionally interrupted by at least one heteroatom chosen from N, O and S and which are optionally substituted by at least one substituent, and
- R3 is chosen from a hydrogen atom, alkyl, alkenyl, alkynyl, cycloalkyl, cycloalkenyl and cycloalkynyl groups and aromatic groups, the hydrocarbon chain of which is optionally interrupted by at least one heteroatom chosen from N, O and S and which are optionally substituted by at least one substituent, which substituent does not comprise an aldehyde CHO.

7. The process as claimed in claim 6, in which R2 is a linear alkyl group, the hydrocarbon chain of which is optionally interrupted by at least one heteroatom chosen from N, O and S and which is optionally substituted by at least one substituent, and R3 is a hydrogen atom.

8. The process as claimed in one of claims 1 to 7, in which the amine is chosen from the compounds 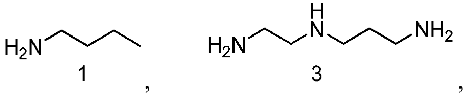 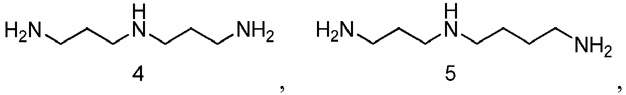 ethylenediamine 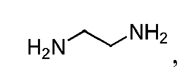 tris(2-aminoethyl)amine 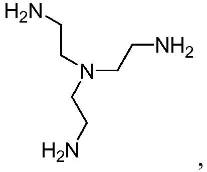 diethylenetetramine 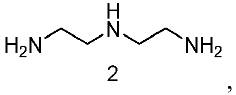 triethylenetetramine 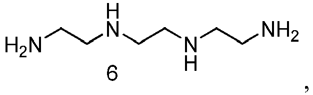 piperazine 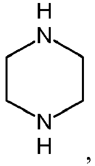 cis-1,4-cyclohexanediamine 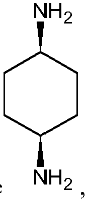 trans-1,4-cyclohexanediamine 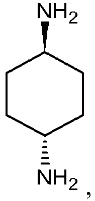 and phenylenediamine

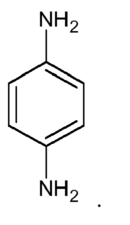

9. The process as claimed in any one of claims 1 to 8, which comprises bringing at least one amine and CO₂, or an adduct formed by bringing an amine and CO₂ into contact, into contact with at least one of said chemical elements.

10. The process as claimed in any one of claims 1 to 9, in which the process is carried out under dynamic combinatorial chemistry conditions.

11. The process as claimed in any one of claims 1 to 10, additionally comprising a stage of observation of the formation of a precipitate.

12. A process for releasing at least one chemical element chosen from poor metals, alkali metals, alkaline earth metals, actinide metals and rare earth metals which comprises the separation, at least partial, preferably total, of the components of an assembly formed by bringing:
- at least one amine and at least one aldehyde and/or one imine (formed by bringing an aldehyde and an amine into contact) and/or CO₂, or
- an adduct formed by bringing an amine and CO₂ into contact,
into contact with a sample comprising at least one of said chemical elements.

13. The process for releasing at least one chemical element as claimed in claim 12, in which the separation of the components is carried out by heating and/or by hydrolysis.

14. The process for releasing at least one chemical element as claimed in claim 12 or 13, in which the separation makes it possible to release and/or reform at least one component, preferably all the components, from the aldehydes, imines, amines and/or CO₂ forming the assembly, or one of their derivatives.

15. A kit for detecting, capturing and/or releasing at least one chemical element chosen from poor metals, alkali metals, alkaline earth metals, actinide metals and rare earth metals which comprises at least one amine and at least one aldehyde and/or one imine and/or CO₂, or an adduct formed by bringing an amine and CO₂ into contact, in which the kit is provided in the form of a support appropriate for use in dynamic combinatorial chemistry and in which the amine and the aldehyde are as defined in one of claims 1 to 8.
